Europäisches Patentamt

European Patent Office

Office européen des brevets

(11) Publication number: **0 221 768**
A2

# EUROPEAN PATENT APPLICATION

(21) Application number: 86308430.7

(22) Date of filing: 29.10.86

(51) Int. Cl.⁴: **C 12 Q 1/02**
G 01 N 33/53
//G01N33/84, G01N33/542

(30) Priority: 29.10.85 US 792433

(43) Date of publication of application:
13.05.87 Bulletin 87/20

(84) Designated Contracting States:
CH DE FR GB IT LI SE

(71) Applicant: DANA-FARBER CANCER INSTITUTE, INC.
44 Binney Street
Boston Massachusetts 02115(US)

(72) Inventor: Reinherz, Ellis L.
113 South Great Road
Lincoln Massachusetts 01773(US)

(72) Inventor: Alcover, Andres
General Ibanes No. 1
Madrid 28003(ES)

(72) Inventor: Weiss, Michael J.
194 Lancaster Terrace
Brookline Massachusetts 02146(US)

(74) Representative: Sheard, Andrew Gregory et al,
Kilburn & Strode 30, John Street
London WC1N 2DD(GB)

(54) Detection of T-cell activation.

(57) A method for detecting activated T-cells in a population of cells comprises determining intracellular calcium ion concentration or intracellular pH in said cells, a calcium ion concentration two fold or greater above that of resting T-cells or a pH increase of over 0.06 pH units above that of resting T-cells indicating activation.

EP 0 221 768 A2

## DETECTION OF T-CELL ACTIVATION

This invention relates to the detection of activated human T-lymphocytes ("T-cells").

Mature human T-cells can exist in a non-activated "resting" state, or in an activated state. Human T-cells can be activated by either of two lineage-specific surface components: the antigen/MHC histocompatibility receptor complex (T3-Ti), and the unrelated T11 molecule. The antigen/MHC receptor complex is comprised of two clonally unique polypeptide chains, termed alpha and beta, which form the putative binding site for antigen/MHC and three noncovalently associated 20-25KD monomorphic T3 molecules thought to be involved in signal transduction. The second structure, the 50KD sheep erythrocyte binding protein, appears to be phylogenetically and ontogenetically more conserved. Although the natural ligand of the T11 system has not yet been identified, monoclonal antibodies to two sets of epitopes, $T11_2$ and $T11_3$ (and to a lesser extent, $T11_1$ and $T11_3$), in concert induce T-cell activation.

T-cells are activated _in vivo_ in response to a variety of stimuli, including infectious agents and sources of foreign antigens, e.g., allografts. The capacity of a patient's T-cells to be activated via the T3-Ti and T11 pathways is an important indication of the status of the patient's immune system. In addition, activation of a patient's T-cells in response to contact with cells from a potential donor of an organ such as a kidney or heart, or of cells, e.g., bone marrow cells, is an indication of histoincompatibility which would lead to rejection of the allograft. Similarly, activation of the donor's T-cells indicates probable

- 2 -

graft-versus-host disease.  Because of the risk of these often fatal developments, histocompatibility typing (known as MHC typing) must be carried out prior to organ or tissue transplantation procedures, often delaying the procedure for several days.

## Summary of the Invention

The invention takes advantage of the discovery that, within minutes of activation via either the T3-Ti or the T11 pathway, there is a large increase in intracellular pH caused by an outflux of hydrogen ions. The invention thus features, generally, a method of detecting activated T-cells in a population of cells by determining intracellular pH in the cells, a pH increase of over 0.06 units indicating activation.  Increased pH is detected by bringing the cytoplasm of the cells in contact with a reporter substance, e.g., a fluorophore, which undergoes a detectable change when exposed to increased pH.  Where a fluorophore is used, it is preferably one which, when exposed to increased pH, emits or excites at a wavelength different from the wavelength at which the fluorophore emits or excites when not exposed to increased pH, or has an altered quantum efficiency (i.e., emits at a greater or decreased intensity) when so exposed.  The method can be used to evaluate a patient's immune status quickly, accurately, and with a high degree of automation.

Where a patient's immune status is evaluated using the method, the method involves, prior to pH measurement, contacting the cells being assayed (generally, peripheral blood cells) with a substance (e.g., an appropriate antibody) which is capable of activating normal T-cells via either the T11 or the T3-Ti activation pathway.  The response (activation or non-activation) of the cells to the stimuli provides

- 3 -

valuable diagnostic information on the immune status of the patient. Anti-Ti antibodies are described in U.S. Pat. No. 4,550,086, hereby incorporated by reference. Anti-T3 antibodies are commercially available from Ortho Pharmaceuticals.

The invention also provides a method for detecting activation of a sub-population of T-cells in a population of cells, e.g., to carry out MHC typing. This method takes advantage of the fact that, within minutes of activation via either the T3-Ti or the T11 pathway, there is, in addition to a pH increase, a large increase in intracellular T-cell calcium ion concentration, which can be determined by bringing the cytoplasm of the cells in contact with a reporter substance e.g., a fluorophore such as indo-1, which undergoes a detectable change when complexed with calcium ions, and detecting the detectable change as an indication of activation. Where a fluorophore is used, it is preferably one which, when complexed with calcium ions, emits or excites at a wavelength different from the wavelength at which the fluorophore emits or excites when not complexed with calcium ions, or has an altered quantum efficiency (i.e., emits at a greater or decreased intensity) when so complexed. According to the method, the T-cell sub-population in which activation is to be detected, e.g., donor or recipient peripheral blood cells, or cells of both donor and recipient, are labeled prior to calcium concentration or pH determination. The cells which are labeled can either be the cells in which activation is to be detected (i.e., the donor's cells when graft-versus-host disease is being tested for, and the recipient's cells when allograft rejection is being tested for), or the cells not expected to be activated; in either case, the

- 4 -

labeling serves to identify the source of the activated cells.  Labeling can be carried out using any of a variety of cellular dyes, e.g., fluorescent DNA dyes such as the DNA dyes sold by Hoechst.  Alternatively, the label can be a reporter group such as a fluorophore coupled to an antibody which binds to T-cells, but does not participate in activation, e.g., anti-Tl or anti-Tl2 antibodies.  Anti-Tl2 antibodies are described in U.S. Pat. No. 4,443,427, hereby incorporated by reference.

Where donor and/or recipient cells are prelabeled with a fluorophore, and activation is detected using a fluorophore, the method preferably emplöys a flow cytometer containing two lasers, one of which illuminates the activation detecting fluorophore, and the other of which illuminates the labeling fluorophore; light of a predetermined wavelength emitted by each is measured, providing both a measure of T-cell activation, and an identification of the source of the activated cells.

Other features and advantages of the invention will be apparent from the following description of the preferred embodiments thereof, and from the claims.

Description of the Preferred Embodiments

The drawings are first described.

Drawings

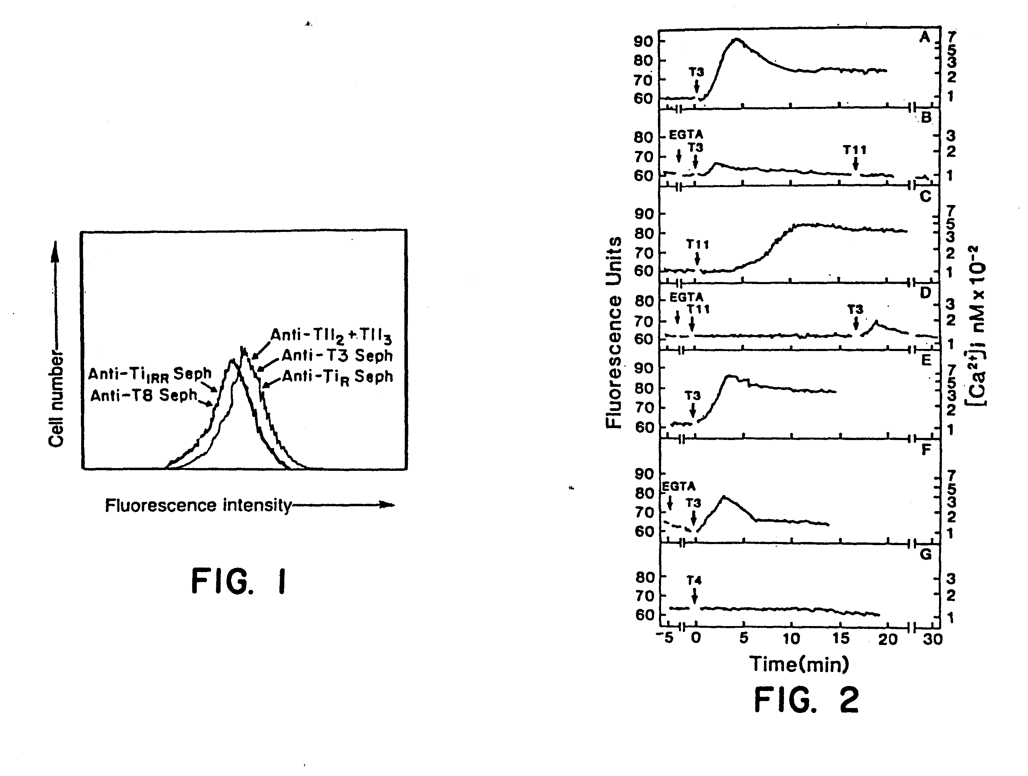

Figs. 1 and 2 are graphs showing an increase in intracellular Ca ions in activated T-cells, as measured using fluorescence.

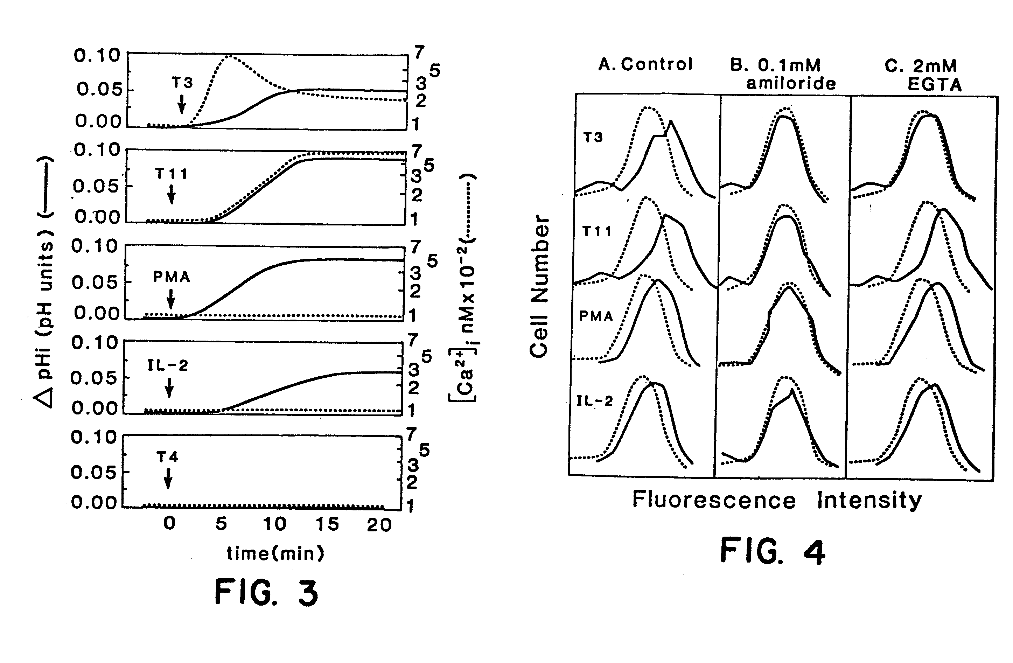

Fig. 3 is a set of panels showing variation in pHi and $[Ca^{2+}]_i$ during T-cell activation.  Changes in fluorescence intensity of either BCECF (solid line) or quin-2 (dashed line) loaded lymphocytes of the helper T cell clone AC3 were measured by spectrofluorimetry after the addition of anti-T3, anti-Tll$_2$, + anti-Tll$_3$, anti-T4 monoclonal antibodies, PMA, or purified 4U/ml purified IL-2.

Fig. 4 is a set of panels showing changes in pH$_i$ analyzed by flow cytometry. Alterations in fluorescence intensity displayed by BCECF loaded cells of the helper clone AC3 were measured by flow cytometry. The curves represent cell number (ordinate) versus fluorescence intensity on a logarithmic scale (abscissa). 10,000 cells were analyzed 1 h after the addition of each stimulus in the presence of control (A), 0.1 mm amiloride (B) or 2 mM EGTA (C). Anti-T3 (IgM) and anti-Tll$_2$ + anti-Tll$_3$ (solid lines) were compared with cells treated with the same concentration of an anti-T4 (19Thy5D7) monoclonal antibody as control (dashed lines) whereas IL-2 and PMA (solid lines) were compared with medium (dashed lines).

Measurement of Intracellular Calcium$^{++}$

The following description of the measurement of intracellular calcium is taken from Weiss et al. (1984) P.N.A.S. U.S.A. 81, 6836-6840, and is presented here to aid in the understanding of the invention.

Lymphocytes were obtained by Ficoll-Hypaque density centrifugation from healthy donors, and T cells were separated as described in Meuer et al. (1983) Science 222, 1239. Contaminating macrophages were rigorously depleted with anti-Mo2 and complement treatment. Indirect immunofluorescence analysis of the residual population demonstrated no detectable macrophages (<0.5% anti-Mol or anti-Mo2 reactive cells). Human T-cell clones were derived from a single donor and propagated in culture as described in Meuer et al., id.

- 6 -

One such clone, a suppressor clone designated 9H5, was loaded with quin-2 acetoxymethylester ("quin-2") a fluorescent indicator of calcium ions, essentially as described in Tsien et al. (1982) J. Cell Biol. 94, 925, as follows. Quin-2 (10mM) (Calbiochem) in dimethyl sulfoxide was added to $10 \times 10^6$ T-cells per ml to a final concentration of 20 μM in RPMI 1640 medium containing 2.5% serum. After a 45-min incubation at 37°C, cells were centrifuged at 200 x g for 10 min, resuspended in the same medium but without quin-2, and incubated for an additional 45 min at 37°C. Cells were then centrifuged and resuspended at $2 \times 10^6$ cells per ml and used immediately.

Monoclonal antibodies used to activate the cells (anti-T3, anti-T8, anti-Tll$_2$, and anti-Tll$_3$) were produced and screened as described in Meuer et al., id, and used in ascites form. An additional monoclonal antibody directed at the 90-kDA Ti receptor structure on one of the isolated clones (AC3) was generated by standard hybridization techniques after immunization of a BALB/cJ mouse with AC3 cells, as described in Kohler and Milstein (1975) Nature 256, 495. Hybridoma supernatants were screened by means of indirect immunofluorescence on an Epics V cell sorter (Coulter). One antibody, termed anti-Ti$_6$, which reacted only with AC3 and none of 20 additional clones from the same donor or resting peripheral T cells (autologous or allogeneic), was cloned by limiting dilution and injected into pristane primed BALB/cJ mice. The resulting ascites fluid was used as a source of antibody and precipitated a disulfide-linked 90-kDA receptor.

Antibodies were purified by protein A-Sepharose, DEAE-Sepharose, or Sepharose G-200 column chromatography and then covalently linked to CNBr-activated Sepharose 4B (Pharmacia). All Sepharose-coupled reagents were tested for functional integrity by immunoprecipitation from $^{125}$I surface labeled cells of the appropriate specificity.

Cells were stimulated with either anti-T3 Sepharose, anti-Ti Sepharose, anti-T8 Sepharose, or a combination of soluble anti-Tll$_2$ and anti-Tll$_3$ antibodies for 30 min at 37$^{\circ}$C, and were then examined using an Epics V dual laser flow cytometer (Coulter). A 5-W argon-ion laser (Spectra-Phycics, Mountain View, CA, model 164-05) with ultraviolet light output capabilities was used to generate forward light scatter and fluorescence measurements. A suspension of quin-2-loaded cells was injected into a laminar saline stream at a rate of 1.0-1.5 x 10$^3$ cells per sec. A focused beam of ultraviolet laser light (180 mW; multiline, 351.1-363.8nm) intersected the saline stream as cells flowed through in single file. Unwanted light scatter and fluorescence were eliminated by use of obscuration bars from both scatter and fluorescence detectors. A 408-nm long pass interference filter and a 530-nm short pass interference filter were placed in front of a photomultiplier tube with a detection range between 300 and 720 nm, peaking at 430 nm. Logarithmic and linear integrated signals generated from the stream-laser intersection were collected and organized in the form of 256 channel single-parameter histograms. Scatter-gated viable cells (1 x 10$^4$) were analyzed, and the resuting histograms provided information regarding percentage reactivity, peak channel location, and relative fluorescence intensity. As shown in Fig. 1, for the representative 9H5 clone, stimulation by anti-T8 Sepharose did not increase cellular fluorescence over background control (anti-Ti$_{IRR}$ Sepharose). In contrast, anti-Ti$_R$ Sepharose, anti-T3 Sepharose, or soluble anti-Tll$_2$ and anti-Tll$_3$ resulted in an easily detectable shift in mean log fluorescence (channel 95 to channel 110). Moreover, the extent of the fluorescence increase with the latter three stimuli was identical. No further increase in quin-2 fluorescence was observed for any stimulus, even up to 6 hrs. after triggering.

Fluorescence was also recorded using a Hitachi Perkin Elmer MPF-2a spectrofluorimeter (Norwalk, CT), excitation at 339 nm (3-nm slit), and emission at 492 nm (11-nm slit), sensitivity of 6. Cells loaded with quin-2 as described above were suspended at $6 \times 10^6$ cells per ml in RPMI 1640 medium without phenol red or vitamins, with 2.5% serum and 10 mM Hepes (pH 7.4). Cells (1.5 ml) were placed in 1-cm quartz cuvettes with a cuvette magnetic stirring bar (Fisher) and were stirred continuously from above. Changes in intracellular calcium activity, which is related to $[Ca^{2+}]_i$, were quantitated by the method of Tsien et al., _id_. At the end of the experiment, Triton X-100 (1%) was added, followed by an excess of EGTA. The $F_{max}$ was taken to be that obtained after addition of Triton X-100 (75 arbitrary units) and $F_{min}$ was that obtained after EGTA (34 a.u.). The fluorescence was 46 a.u. before and 65 a.u. after anti-Tll$_2$ and anti-Tll$_3$ addition. Using a $K_d$ of $115 \times 10^{-9}$M, this corresponds to a change in $[Ca^{2+}]_i$ from ≈50 to $400 \times 10^{-9}$M.

Fig. 2 shows a recording of a representative experiment in which quin-2 loaded cells from a helper T-cell clone (AC3) were stimulated with anti-T3 (A,B,E,F) or anti-Tll$_2$ + anti-Tll$_3$ (C,D). In B, D, and F, 3mM EGTA (final concentration) was added to the media 5 min prior to addition of the stimuli. Response of cells activated with anti-T4 antibody is shown in panel G. Fluorescence was determined by spectrofluorimetry as described above with quin-2 loaded cells. The results are representative of 10 assays performed on human T-Cell clones and the tumor T-cell line, REX. As shown, within 2 min of stimulation with anti-T3 or anti-Tll$_2$ + anti-Tll$_3$, quin-2 fluorescence began increasing, reflecting an increase in $[Ca^{2+}]_i$. The fluorescence increased for $\approx$10 min before reaching a new steady state. This increase in fluorescence reflected an increase in average $[Ca^{2+}]_i$ from $\approx$50 to 400 x $10^{-9}$M. In contrast, even a large excess of purified IL-2 (3 units/ml) did not cause any change in quin-2 fluorescence (data not shown). As shown in panels B and D, in the presence of EGTA, the addition of anti-Tll$_2$ and anti-Tll$_3$ did not result in an increase in quin-2 fluorescence.

The above experiments show that there is a rapid increase in the intracellular $Ca^{2+}$ concentration after triggering of either pathway by specific monoclonal antibodies directed at their various components. $Ca^{2+}$ is thus an important early actor in the physiologic stimulation of human T-cell clones derived from inducer, suppressor, and cytotoxic subclasses. In addition, it was demonstrated that flow cytometry, as opposed to spectrofluorimetric techniques that take an average of the sample, affords a convenient way to examine changes in the fluorescence of discrete populations of cells. This is especially valuable when dealing with polyclonal population of lymphocytes that may exhibit several levels of quin-2 fluorescence and differing responses to a given stimulus.

- 10 -

Simultaneous Measurement of Two Fluorophores

In the following experiments, describing methods of the present invention, in addition to quin-2, the fluorescent dyes indo-1, fura-2, diethyloxadicarbocyanine ($DiOC_2(5)$), and phycoerythrin were used. Phycoerythrin was conjugated to anti-T11$_3$ antibody as follows. 1.0 mg of protein A purified anti-T11$_3$ antibody was reacted with 0.5 mg of R-phycoerythrin (pyridyldisulfide derivative) as described in Oi et al. (1982) J. Cell Bio. 93, 981. Anti-T3 (2Ad2) phycoerythrin was obtained from Coulter Immunology (Hialeah, FL).

Cells were loaded with 10ug/ml indo-1 as described above for quin-2 and washed as described for quin-2 using a final concentration for the assay of $10^6$ cells/ml. After indo-1 loading, cells were incubated for 60 min with 2 nM $DiOC_2(5)$. Measurement of indo-1 fluorescence by flow cytometry was performed using an Epics V dual laser flow cytometer (Coulter Electronics, Hialeah, FL). A 5W argon ion laser (Spectra-Physics, Mountain View, CA, model 164-05) with ultraviolet light output capabilities was used to generate forward angle light scatter and fluorescence measurements. A single cell suspension of indo-1 loaded cells was injected into a laminar saline stream at a rate of 1.0-1.5 x $10^3$ cells/second. A focused laser beam of ultraviolet light (50 mW multi-line 351.1-363.8mm) intersected the saline stream as cells flowed through in single file order. Unwanted light scatter and fluorescence were elimnated by used of obscuration bars in front of detectors from both scatter and fluorescence. The measurement of indo-1 fluorescence was achieved by using a 408 long pass and a 530 short pass filter combination directly in front of a photomultiplier tube which had a spectral response ranging from 300-700 nm with peak sensitivity at 430 nm.

Data was acquired in either single or two parameter histogram format. For single parameter analysis, logarithmic and linear integrated fluorescence signals generated from single cells intersecting the laser beam were collected and organized in the form of 256 channel histograms. Fluorescence of viable cells, as determined by forward angle light scatter, as analyzed and histograms were generated, providing information as to the percentage reactivity, peak channel location and relative fluorescence intensity. Two parameter 64 x 64 channel histograms were constructed with time versus log or linear integrated fluorescence along the X and Y axis, respectively. Fluorescence measurements were recorded every 16 sec over a 15 min. period. Baseline fluorescence was established for about 120 sec prior to addition of the stimuli. Measurements were resumed while the sample was gently stirred.

Simultaneous measurements of both indo-1 and $DiOC_2$ (5) fluorescence or indo-1 and phycoerythrin surface labeled antibody fluorescence was achieved by employing a second laser beam, focused approximately 70 u downstream from the UV laser beam. The second beam was a 200 MW, 568.2 nm laser line derived from a krypton ion laser (Spectra-Physics). Forward angle light scatter and indo-1 derived fluorescence signals were delayed in time 7 micro sec until fluorescence signals were generated from the 568.2 nm laser line beam. Spectral separation of either indo-1 fluorescence emission and $DiOC_2$(5) dye emission or indo-1 and phycoerythrin fluorescence was accomplished by use of a 590 dichroic filter which reflected longer wavelength light to a photomultiplier tube which had a 595 nm as well as a 610 nm interference filter. Shorter wavelength emission was transmitted through the dichroic filter and the 408 nm long pass/530 nm short pass combination filters were placed in front of the photomultiplier tube responsible for measurement of indo-1 fluorescence. In addition, a 530 short pass filter was placed in front of the scatter sensor to eliminate unwanted light from the 568.2 nm laser line.

Control samples were analyzed to assure minimal spectral emission overlap as well as optimal fluorescence detection. These control samples consisted of cells loaded with no dye, one dye, or combinations.

Dual fluorescence flow cytometric analysis of T-cells activated via T3-Ti or Tll was carried out as folows. Phycoerythrin labeled anti-T3 monoclonal antibody (2Ad2) or unlabeled anti-Tll$_2$ plus phycoerythrin labeled anti-Tll$_3$ were used to trigger the helper T-cell clone 9H5B. Green (indo-1) and red (phycoerythrin) fluorescence were monitored simultaneously as described above.

Rather than using increase in intracellular Ca$^{++}$ as a measure of activation, increase in intracellular pH can be measured in an analogous fashion. Cells are loaded with a pH-sensitive substance, e.g., a fluorophore whose emission wavelength changes when pH increases. One such fluorophore is 2', 7'-bis (2 carboxyethyl) -5 (and 6) carboxy fluorescein acetoximethyl ester, available from Molecular Probes, Junction City, Oregon. T-cell activation causes a pH increase of 0.04 to 0.08 units (typically 0.06 units), from about 7.0 to 7.06. In more detail, such a procedure was carried out as follows.

Human T cell helper clones AC3 or 9H5B or the cytotoxic clone J were loaded with bis-carboxyethyl carboxy fluorescein (BCECF) by incubating with 2 mM of the acetoxymethyl ester BCECF-AM (Molecular Probes, Junction City, OR) for 1 h at 37°C in 2.5% fetal calf serum (FCS) wash (minimum essential medium, 1% penicillin-streptomycin, 10mM HEPES buffer, pH 7.0). The cells were washed in the same medium and resuspended in final medium ($HCO_3$-free, RPMI 1640 balanced salt solution, 10mM HEPES buffer pH 7.0). $Na^+$ free media were prepared by substituting NaCl by equiosmolar concentrations of choline chloride. Spectrofluorimetric measurements were performed according to Rink et al. J. Cell Biol. (1982) 95, 189 in a Hitachi-Perkin Elmer MPF-2A fluorimeter at an exitation wavelength of 470 nm and emission wavelength of 530 nm. Monoclonal antibodies against T3 (2Ad2, IgM isotype) or the combination of two anti-T11 monoclonal anti-T11$_2$ (IgG2) and anti-T11$_3$ (IgG3) antibodies were used to trigger T3 or T11 pathways of activation, respectively at a 1:200 dilution of ascites. PMA (Sigma, St. Louis, MO) at 100 ng/ml final concentration or 4 U/ml of purified IL-2 were also used. Monoclonal antibodies against T4 (19Thy507, IgG2 isotype or 7T4-6Cl, IgM isotype) or the same volume of solution were used as control. Titration of BCECF fluorescence vs. pH was carried out after lysing the cells with NP-40 detergent.

Flow cytometric measurements were performed before, during, and after activation with the monoclonal antibodies specific for the surface T3-Ti complex and T11 structures on an Epics V flow cytometer (Coulter Electronics, Hialeah, FL) with coherent innova 90-5 Argon laser. The exitation wavelength was 476 nm, power 300 mW and the fluorescence filters were 488 nm long pass interference filter and 495 and 515 nm long pass absorption filters. Fluorescence high voltage 370 dial setting (540 volts). The cells to analyze were loaded with BCECF as described above. 10,000 cells were analyzed for each sample and fluorescence (abscissa) intensity determined on a logarithmic scale 1 h after the addition of the appropriate stimuli. The concentration of the stimuli were the same as in the spectrofluorimetric measurements.

As shown in Fig. 3, for the representative helper T cell clone AC3, a detectable rise in pHi (0.06-0.08 pH units) was observed utilizing BCECF and fluorescence spectrofluorimetry (solid line) after triggering through T3-Ti or T11 pathways as well as upon stimulation with PMA or Il-2. pH changes were noted within several minutes of addition of various stimuli to the T-cells, reached maximal levels after 10 min and persisted for the length of the observation (3 h). pH measurements for longer intervals were not possible owing to the potential cellular metabolic alterations of the pH probe affecting the accuracy of subsequent measurements. While the magnitude of these changes with clonal physiologic T-cell populations utilized herein is less than reported by others, it is consistently reproducible. Furthermore, these pHi alterations are not a non-specific effect of surface structure interaction with monoclonal antibodies or ligand since anti-T4 antibodies of either the IgG or IgM class induced no alterations in pHi after binding to the T4 molecule. The ability of phorbol ester to induce pHi change has been noted for various other cell types previously. While changes in pHi in response to anti-T3 antibodies have been recently reported for the tumor T

- 15 -

cell line HPB-ALL (Rosoff et al. (1985) J. Biol. Chem. $\underline{260}$, 14053), the present results extend this observation to normal T-cell populations and indicate that T11 triggering can induce a similar effect.

Although spectrofluorimetry allows accurate detecting of average changes in fluorescence for a given population as a whole, as is mentioned above in connection with $Ca^{++}$ measurements, it cannot provide information about alterations at the single cell level. To examine the individual cellular response and avoid any confounding effects of fluorescence background resulting from pharmacologic additives in the assay media, we developed a flow cytometric procedure which measures BCECF fluorescence emitted by individual cells. As shown in Fig. 4, and in agreement with the results obtained by spectrofluorimetry, anti-T3, the combination of anti-T11$_2$ + anti-T11$_3$, PMA and IL-2 all induce an increase in BCECF fluorescence of clone AC3. In contrast and as expected, anti-T4 monoclonal antibodies of either IgG or IgM subclass or anti-T11$_1$ or anti-T11$_2$ antibodies alone failed to induce an increase in pHi. The relative fluorescence intensity induced by the various stimuli is also consistent with the data obtained by spectrofluorimetry. Similar results were obtained with another T cell helper clone, 9H5B, the T8+ cytotoxic T cell clone, J, and the tumor T cell line Jurkat. The pHi changes in response to the different stimuli were not induced when the cells were assayed in the presence of amiloride, a $Na^+/H^+$ antiport which exchanges extracellular sodium for intracellular protons. Thus, the $Na^+/H^+$ exchanger is implicated in the regulation of pHi in human T cells as well as a variety of cell types previously described. Furthermore, these data and the results in Fig. 4 imply that while protein kinase C activation induces $Na^+/H^+$ exchanger function, it does not by itself lead to the opening of transmembrane calcium channels.

## Other Embodiments

Other embodiments are within the following claims. For example, reporter substances and labels other than flurophores, and detection methods other than laser-based methods, can be used. Cells can be loaded with chromophores which change color in the presence of calcium ions, for example, and the color change detected spectro-photometrically. Another alternate method would be to employ a dye whose optical density increases or decreases in the presence of calcium ions. Labeling of cells for identification can also be carried out using chromophores, as well as radioisotopes.

## CLAIMS

1. A method for detecting activated T-cells in a population of cells, said method comprising determining intracellular calcium ion concentration or intracellular pH in said cells, a calcium ion concentration two fold or greater above that of resting T-cells or a pH increase of over 0.06 pH units above that of resting T-cells indicating activation.

2. A method as claimed in claim 1 wherein said intracellular pH determination is carried out by bringing the cytoplasm of the cells in contact with a reporter substance which undergoes a detectable change when exposed to increased pH, and detecting said detectable change as an indication of said activation.

3. A method of detecting activation of a sub-population of T-cells in a population of cells, said method comprising

labelling said sub-population of T-cells with a detectable label, and

determining intracellular calcium ion concentration or intracellular pH in said labelled sub-population, a calcium ion concentration two fold or greater above that of resting T-cells, or a pH increase of over 0.06 pH units, indicating activation.

4. A method as claimed in claim 3 wherein said labeling comprises binding to said cells an antibody conjugated with a reporter group.

5. A method as claimed in claim 4 wherein said antibody does not cause activation of said cells to which it binds.

6. A method as claimed in claim 3, 4 or 5 wherein said method further comprises detecting said labeled cells.

7. A method as claimed in claim 6 wherein said reporter substance and said detectable label comprise, respectively, a first and second fluorophore,

said detectable change indicative of activation is detected by illuminating said cells with a first laser and measuring light of a predetermined wavelength emitted by said first fluorophore, and

said labeled cells are detected by illuminating said cells with a second laser and measuring light of a predetermined wavelength emitted by said second fluorophore.

8. A method as claimed in claim 7 wherein said first fluorophore, when complexed with calcium ions or exposed to increased pH, emits or excites at a wavelength different from the wavelength at which said fluorophore emits or excites when not complexed with calcium ions or not exposed to increased pH, or has an altered quantum efficiency when so complex or exposed.

9. A method as claimed in any one of claims 1 to 8, further comprising prior to said detecting, contacting said cells with a substance capable of activating normal T-cells via the T3-Ti activation pathway.

10. A method as claimed in any one of claims 1 to 8 further comprising, prior to said detecting, contacting said cells with a substance capable of activating normal T-cells via the T11 activation pathway.

11. A method as claimed in claim 9 wherein said activating substance comprises an antibody to the T3 surface receptor.

12. A method as claimed in claim 10 wherein said activating substance comprises an antibody to the T11 surface receptor.

FIG. 2

$[Ca^{2+}]i$ nM $\times 10^{-2}$

Time(min)

Fluorescence Units

FIG. I

Anti-TII$_2$+TII$_3$
Anti-T3 Seph
Anti-Ti$_R$ Seph
Anti-Ti$_{IRR}$ Seph
Anti-T8 Seph

Cell number

Fluorescence intensity

FIG. 3

FIG. 4